# EUROPEAN PATENT APPLICATION

(11) **EP 2 025 686 A2**
(43) Date of publication of application: **18.02.2009**
(21) Application number: 07788612.5
(22) Date of filing: 28.05.2007
(51) Int. Cl.: C07K 16/40, A61K 39/395, C12N 5/18, A61P 35/00, G01N 33/577

(54) **ALPHA ANTICHOLINE KINASE MONOCLONAL ANTIBODIES AND THEIR USE IN ANALYTICAL TECHNIQUES FOR THE DIAGNOSIS OF CANCER AND THE PREPARATION OF MEDICINAL PRODUCTS**

(30) Priority: 29.05.2006 ES 200601407
(71) Applicant: Consejo Superior de Investigaciones Cientificas, 28006 Madrid (ES)
(72) Inventor: RAMIREZ DE MOLINA, Ana, 28029 Madrid (ES); GALLEGO ORTEGA, David, 28029 Madrid (ES); LACAL SANJUAN, Juan Carlos, 28029 Madrid (ES)
(74) Representative: Illescas, Manuel
(86) International application number: PCT/ES2007/000364
(87) International publication number: WO 2007/138143

(57) **Abstract**

Monoclonal antibodies against choline kinase alpha and the use thereof in analytical techniques, cancer diagnosis and drug preparation. Monoclonal antibodies AD1, AD2, AD3, AD4, AD5, AD6, AD7, AD8, AD9, AD10, AD11, AD12, AD13 and AD14 are highly specific and recognise the human choline kinase alpha protein (ChoKα) with a high sensitivity. This allows for them to be used in the detection and/or quantification of this protein in any type of sample, including biological samples. In particular, it allows for the development of *in vitro* methods for the diagnosis of cancer, the prognosis and/or follow-up of its evolution or the evaluation of anti-cancer therapies wherein the expression level of ChoKα is determined by means of one or more of the monoclonal antibodies of the invention or of kits that comprise them. Moreover, AD3, AD8 and AD13, which interfere with ChoKα's choline kinase activity, may be used in the preparation of anti-cancer drugs.

## Description

### FIELD OF THE INVENTION

The invention relates to monoclonal antibodies capable of binding to human choline kinase alpha. Specifically, the invention relates to monoclonal antibodies AD1, AD2, AD3, AD4, AD5, AD6, AD7, AD8, AD9, AD10, AD11, AD12, AD13 and AD14, the hybridomas that produce them, the compositions that contain them, the use thereof to detect and/or quantify the presence of the human choline kinase alpha protein and the methods to conduct *in vitro* diagnoses of the presence of tumours in an individual or predict their potential evolution that use said antibodies.

### BACKGROUND OF THE INVENTION

Choline kinase α (ChoKα) is the first enzyme in the Kennedy pathway for the biosynthesis of phosphatidylcholine (PC), the main phospholipid in biological membranes. There is consistent evidence that demonstrates the involvement of choline kinase and its product, phosphorylcholine (PCho), in the carcinogenesis process (1-4).

ChoK is overexpressed in a high percentage of cell lines derived from human tumours, as well as in different human tumoural tissues, such as breast, lung, colon and prostate (5,6). Taken as a whole, these tumours represent over 70% of the total number of cancer cases in developed countries. The rate of overexpression or increased ChoK activity in these four types of cancer ranges from 40% to 60% (5-7). Recently, the authors of this invention demonstrated not only that the choline kinase alpha protein is increased in the above-mentioned human tumoural tissues (breast, lung, colon and prostate), but that it is also increased in other types of tumours, such as bladder and haematological tumours. Moreover, the overexpression of said enzyme induces tumours *in vivo* and seems to be related to the cancer severity, the stage of development thereof and the decrease in survival time for patients exhibiting tumours when said overexpression is detected. Furthermore, it has been demonstrated that the inhibition of ChoKα in particular, by means of both chemical inhibitors and interference RNA, represents an effective anti-tumour strategy. All this suggests that ChoKα is a new, significant tumour marker, useful for diagnosis, prognosis or response to the treatment. For this reason, international patent application PCT/ES2006/070047 provides a method to detect the presence of cancer in an individual, to determine the stage or the severity of said cancer, or to monitor the effect of the therapy administered to an individual suffering from cancer, by detecting and quantifying the choline kinase alpha protein, as well as detecting and quantifying said enzyme's messenger RNA or the corresponding cDNA.

The procedures designed for the early, reliable diagnosis of cancer provide valuable information that may improve treatment of this disease, contributing to achieve an increase in life expectancy. The methods which comprise determining the overexpression of ChoKα in human tumours may represent a significant advance both in cancer diagnosis and in the identification of potential candidates for therapy using ChoK inhibitors. The development of these methods entails the development of suitable reagents and methodologies which allow determining the overexpression of ChoKα. A suitable tool to accurately determine the increase in ChoKα levels in tissue samples may be monoclonal antibodies, which are populations of antibody molecules, all identical, produced by a single cell clone, which have the same specificity, as opposed to polyclonal antibodies or polysera, which are complex antibody populations, formed by different classes of immunoglobulin molecules with a variety of specificities. These molecules have been highly successful as analytical reagents, because they are chemically well-defined substances, the nature and structure whereof are known in detail; this allows for the formulation of stable preparations and facilitates the procedures designed for their conjugation by means of tracers such as fluorescent substances, enzymes, radioisotopes, colloidal gold, electron-dense molecules (ferritin), etc.; moreover, these reagents may be prepared in pure form, under very controlled conditions and in large quantities. In particular, monoclonal antibodies are widely used in biomedical research and the diagnosis of many diseases, including cancer, and offer high sensitivity and specificity, both of which are necessary for said studies. However, there are no published references about monoclonal antibodies against ChoKα in the State of the Art. There are commercially available antibodies against this protein, all polyclonal, which have a limited specificity and, when used in Western blot immunoassays, lead to many unspecific bands. This low specificity is particularly problematic when it is desired to perform immunohistochemical assays, for which it is important not to have unspecific staining; consequently, there also aren't any published references in the State of the Art which describe immunohistochemical assays wherein the ChoKα protein is detected. For all these reasons, given the proven involvement of ChoKα in human carcinogenesis, there was a need to develop monoclonal antibodies against choline kinase alpha, preferably with a high specificity and sensitivity, with a good capacity to precipitate the choline kinase alpha protein from the solutions it is in or to interact with said protein in tissue samples or solutions it is present in (this allows for the application of immunochemical methods in general and, in particular, immunohistochemical methods, for which until now there were no suitable tools and which would be useful, for example, in the analysis of removed tumours). This would facilitate the evaluation of ChoKα levels and the establishment of relationships between the results obtained and the potential presence of cancer in the analysed samples' source individuals, the determination of the cancer stage or severity, the prognosis of its potential evolution, the follow-up of said evolution or the monitoring of the effect of the therapy administered to an individual suffering from cancer. Said antibodies would be useful, moreover, in any procedure wherein it is desired to detect and/or quantify the choline kinase alpha protein present in any given sample.

### SUMMARY OF THE INVENTION

This invention provides a set of monoclonal antibodies against ChoKα that are useful tools in several molecular biology techniques, including the analysis of biological samples, as well as in immunohistochemical studies, especially those conducted on human tissue samples as a part of diagnostic or prognostic methods for the presence of cancer in said tissue; evidence is provided regarding this potential use. These antibodies constitute a specific tool that allows for the detection of ChoKα without interacting, for example, with another isoenzyme with which it has 70% homology, choline kinase beta. Having a specific reagent available that distinguishes between both isoenzymes made it possible to perform the assays described in international patent application PCT/ES2006/070047, pending publication, which demonstrate that the overexpression of ChoKα, and not that of ChoKβ, is capable of inducing carcinogenesis in human beings; moreover, ChoKα levels are related to the cancer severity and the decrease in survival time for individuals exhibiting tumours with high ChoKα levels. For all these reasons, said international patent provides methods to diagnose the survival time of a patient suffering from cancer as well as methods to monitor the effects of anti-cancer therapies, both of which are based on the evaluation of the expression level of the choline kinase alpha protein by, for example, determining the concentration of said protein in tissue samples taken from the patient. Performing said methods would not be possible with the antibodies known in the state of the art, which are polyclonal and unspecific, and requires the specificity of monoclonal antibodies, the production and characteristics whereof are disclosed in this invention, and the existence and the properties whereof have not been made accessible in any manner to the general public at the time when this specification is submitted.

Therefore, one aspect of the invention comprises monoclonal antibodies AD1, AD2, AD3, AD4, AD5, AD6, AD7, AD8, AD9, AD10, AD11, AD12, AD13 and AD14, which specifically bind to the human choline kinase alpha protein.

A second aspect of the invention are the hybridoma cell lines capable of producing monoclonal antibodies AD1, AD2, AD3, AD4, AD5, AD6, AD7, AD8, AD9, AD10, AD11, AD12, AD13 and AD14.

Another aspect of the invention are those compositions comprising one or more of monoclonal antibodies AD1, AD2, AD3, AD4, AD5, AD6, AD7, AD8, AD9, AD10, AD11, AD12, AD13 and AD14.

An additional aspect of the invention is the use of monoclonal antibodies AD1, AD2, AD3, AD4, AD5, AD6, AD7, AD8, AD9, AD10, AD11, AD12, AD13 and AD14 to detect and/or quantify the presence of human choline kinase alpha in any given sample.

An additional aspect of the invention are cancer *in vitro* diagnostic methods, particularly for breast, lung, colon, prostate and bladder cancer, and haematological tumours, which comprise the detection of the human choline kinase alpha protein present in a biological sample taken from a human being and/or the quantification of the human choline kinase alpha protein by using one or more of monoclonal antibodies AD1, AD2, AD3, AD4, AD5, AD6, AD7, AD8, AD9, AD10, AD11, AD12, AD13 and AD14.

Another, additional aspect of the invention are assay kits for the diagnosis of cancer, the prognosis of its evolution, the follow-up of said evolution or the evaluation of an anti-cancer therapy which comprise at least one of the monoclonal antibodies of the invention: AD1, AD2, AD3, AD4, AD5, AD6, AD7, AD8, AD9, AD10, AD11, AD12, AD13 and AD14.

Finally, another aspect of the invention is the use of at least one monoclonal antibody selected from AD3, AD8 and AD11 for the manufacturing of a drug intended as anti-cancer therapy.

### BRIEF DESCRIPTION OF THE FIGURES

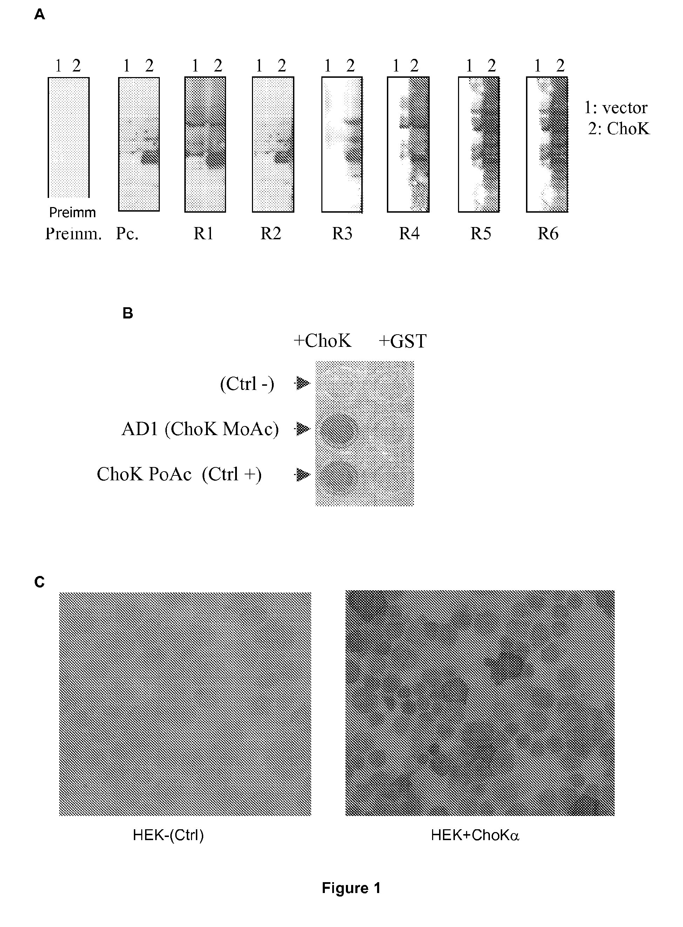

Figure 1 shows the tests conducted during the generation and the selection of the monoclonal antibodies and hybridomas of the invention. Part A shows the Western blot analysis of the sera of immunised mice (M1, M2, M3, M4, M5 and M6), compared to the results obtained with a pre-immune serum (Preimm.) and a polyclonal serum (Polyc.); in all cases, lane 1 corresponds to human HEK293T cell extracts transfected with an empty vector, which does not express ChoKα, as a control, and lane 2 corresponds to HEK293T cells which overexpress ChoKα because they have been transfected with an expression vector of said protein. Part B shows an example of the ELISA assays performed during the selection of the hybridomas, that pertaining to the hybridoma of antibody AD1: the wells on the left-side column were coated with recombinant ChoKα and those on the right were coated with GST and incubated with: (Ctrl.-): anti-GST monoclonal antibody; AD1: supernatant of the culture medium of the hybridoma pertaining to antibody AD1; ChoK PoAC (Ctrl.+): anti-ChoKα polyclonal antibody. Part C shows an example of the confirmation of the selection of hybridomas by means of immunocytochemistry (cytospin) of HEK293 cells transfected with a vector that does not express ChoKα (photo labelled as HEK-Ctrl) or a ChoKα expression vector (photo labelled as HEK+ChoKα) which were subject to an immunostaining process with the culture supernatant of the same hybridoma.

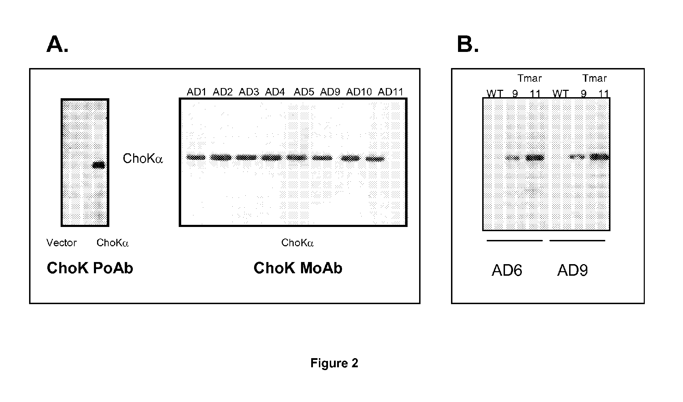

Figure 2 shows an example of the Western blot immunoassays performed with the antibodies of the invention. Part A shows the results obtained upon incubating lysates of HEK293 cells transfected with an empty vector (lane labelled as "Vector") or HEK293 cells transfected with a ChoKα expression vector (lanes labelled as "ChoKα), either with anti-ChoKα polyclonal antibody (photograph labelled as "ChoK PoAb") or with the monoclonal antibody of the invention (photograph labelled as "ChoK MoAb") that is indicated on each lane. Part B shows the results obtained upon incubating lysates of cells that express mouse choline kinase α, NIH3T3-Tmar-9 (lanes labelled with number 9) or NIH3T-Tmar-11 (lanes labelled with number 11), with antibodies AD6 and AD9.

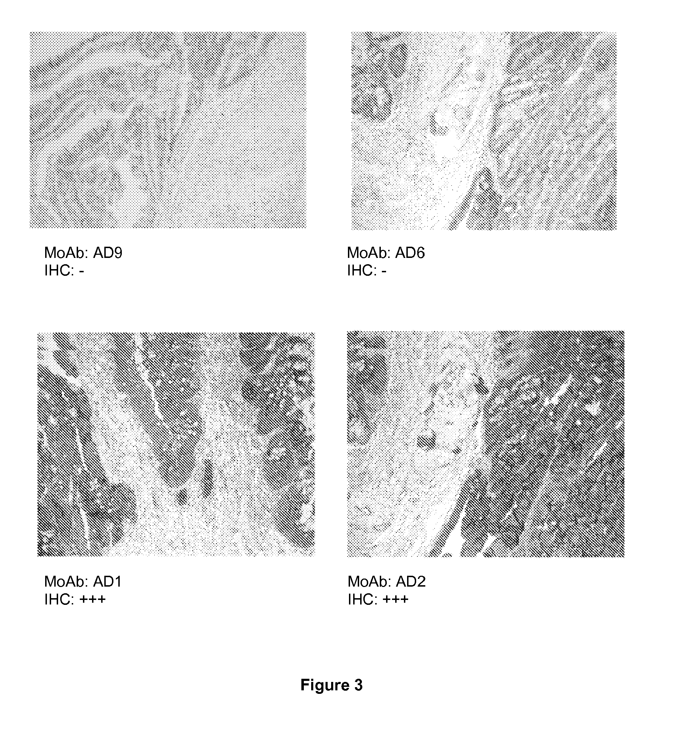

Figure 3 shows examples of negative staining (upper left-hand-corner photograph), weak staining (upper right-hand-corner photograph) and strong staining (lower photographs) of samples taken from colorectal tumours whereon an immunohistochemical analysis (IHQ) with antibodies AD9, AD6, AD1 and AD2 had been performed, as indicated below each photograph.

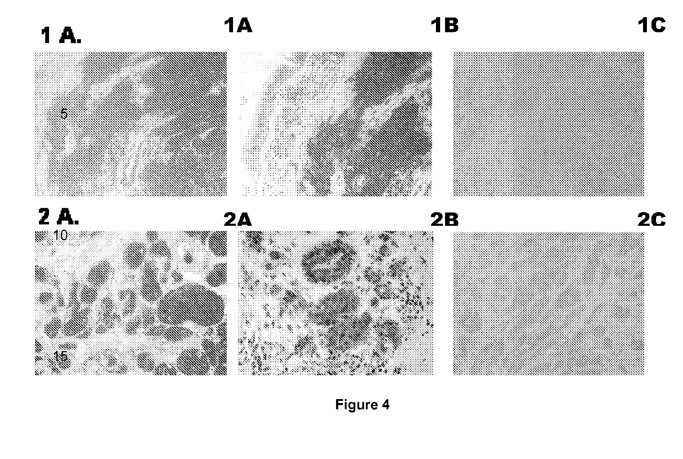

Figure 4 shows the immunohistochemical analysis of lung (1) and breast tumours (2) with representative anti-ChoKα monoclonal antibodies. In each case, the photographs labelled with letters A (1A and 2A) and B (1 Band 2B) correspond to strongly stained tumoural tissues, with a slight baseline staining of the adjacent normal tissues. The photographs labelled with letter C (1 C and 2C) correspond to samples of both types of tumours for which staining with these immunohistochemically useful antibodies was negative. Contrast staining was performed with haematoxylin.

### DETAILED DESCRIPTION OF THE INVENTION

The invention relates to monoclonal antibodies AD1, AD2, AD3, AD4, AD5, AD6, AD7, AD8, AD9, AD10, AD11, AD12, AD13 and AD14, which are capable of specifically binding to the choline kinase alpha protein (ChoKα), an enzyme that is involved in the synthesis of phospholipids and which has been found to be involved in cell proliferation, the transformation and the progression of tumours. In particular, its overexpression seems to be involved in the carcinogenesis of breast, lung, colon and prostate tumours in human beings, which suggests that it is a potential tumour marker (5,6). The detection and quantification of its levels, however, requires the development of suitable tools to do so; these may be monoclonal antibodies, especially those that exhibit a high specificity and sensitivity towards this antigen, ChoKα.

To this end, the invention provides monoclonal antibodies AD1, AD2, AD3, AD4, AD5, AD6, AD7, AD8, AD9, AD10, AD11, AD12, AD13 and AD14, the specificity and sensitivity whereof are demonstrated in the Examples described further below. Thus, for example, it is observed that, although there is a 77% structural similarity between human and mouse choline kinase (19), only monoclonal antibodies AD4 and AD10 effectively bind to mouse choline kinase (mChoK) in an immunoassay performed on proteins transferred to nitrocellulose membranes, which shows the high specificity of the monoclonal antibodies of the invention, especially AD1, AD2, AD3, AD5, AD6, AD7, AD8, AD9, AD10, AD11, AD12, AD13 and AD14. The corresponding hybridomas capable of producing each of said antibodies are also provided, which makes it possible to produce them in large quantities and facilitates the purification thereof.

The monoclonal antibodies of the invention constitute a useful, reliable tool to be used in basic assay techniques, such as immunoassays performed on proteins transferred to membranes (a technique known as Western blot and abbreviated as WB) and immunoprecipitation (IP); therefore, they may be excellent tools to increase knowledge about ChoKα's biology or for any type of assay wherein it is desired to check the presence of said protein and/or quantify it. Consequently, the scope of the invention comprises the use of one or more monoclonal antibodies selected from the group formed by AD1, AD2, AD3, AD4, AD5, AD6, AD7, AD8, AD9, AD10, AD11, AD12, AD13 and AD14 for the detection and/or quantification of the kinase alpha protein in any given sample. There is special preference for biological samples, these being understood to mean any samples taken from a human being, either from a tissue therefrom or from any of the so-called biological fluids, the most typical being urine, blood and the plasma obtained therefrom, although this term also comprises any other body fluid, such as cerebrospinal liquid or synovial liquid. The technique used for the detection and/or quantification of the human choline kinase alpha protein by means of one or more monoclonal antibodies of the invention may be any of the techniques known by those skilled in the art, which include those that use Western blots, immunoprecipitation or, in the case of tissues, immunohistochemical techniques; specific assays of these with the monoclonal antibodies of the invention are also described in the Examples shown further below.

The immunoprecipitation assays performed to characterise the antibodies of the invention have revealed that these antibodies do not recognise the same epitopes, since some of them seem to interfere with enzymatic activity and it is not possible to detect choline kinase enzymatic activity starting from choline kinase alpha proteins immunoprecipitated with some of the monoclonal antibodies of the invention, while others (AD1, AD2, AD4, AD5, AD7, AD13 and AD14) do not prevent the immunoprecipitated proteins from exhibiting enzymatic activity when they are provided with the suitable substrate and the suitable conditions. For this reason, these last antibodies (AD1, AD2, AD4, AD5, AD7, AD13 and AD14) are those preferred to be used in any assay that involves immunoprecipitation of choline kinase alpha in such a way that said protein maintains its choline kinase activity, especially AD1, AD4, AD5, AD7 and AD13, which lead to a greater degree of enzymatic activity in the inmunoprecipitates generated thereby.

Immunohistochemical studies, a technique that is intended to detect and identify *in situ* biomolecular components that are an integral part of cells and tissues, are another clinically-relevant technique in which monoclonal antibodies are useful. This technique may be very useful for the *in vitro* diagnosis of cancer in tissue samples taken from patients, by assaying a specific marker whose association with cancer is known. As mentioned, studies indicate that ChoKα represents a new, significant tumour marker, the expression whereof is increased in a significant percentage of tumours, specifically by 40-60% in breast, colon, lung, prostate, bladder or haematological tumours; this enzyme's capacity, when overexpressed, for the *in vivo* induction of tumours and the effectiveness of its inhibition as an anti-tumour strategy have also been proven. Moreover, a relationship has been observed between its expression level and the severity of the tumours. For all these reasons, the scope of the invention comprises not only the use of the monoclonal antibodies of the invention for the detection and/or quantification of the ChoKα protein by means of immunohistochemical techniques, preferably applied to tissue samples taken from human beings, especially breast, colon, lung or bladder tissue samples, but also includes, in addition, any method for the diagnosis of cancer, the prognosis of its evolution, the follow-up of said evolution or the evaluation of the response to a treatment in which the ChoKα protein is detected and/or quantified by means of the monoclonal antibodies of the invention. These methods' potential and the capacity of the monoclonal antibodies of the invention to be useful in performing them is shown in the examples described further below, in which immunohistochemical assays were performed on samples taken from the biopsies of patients who had previously been diagnosed with breast or lung cancer. In most cases, this technique made it possible to clearly distinguish between the tumoural tissue wherein ChoKα is overexpressed (which appeared as strongly labelled) from the surrounding normal tissue, wherein staining with the anti-ChoKα antibodies was only faint. As has been discussed, the previous biochemical data on ChoKα's overexpression and increased activity in human tumours demonstrated that there is a 40%-60% incidence of ChoKα in different types of human cancer (5-7). Therefore, baseline staining is expected in those tumours wherein the ChoKα levels are similar to those of the surrounding normal cells. Consequently, the data obtained in the immunohistochemical tests performed with the anti-ChoKα monoclonal antibodies of the invention are consistent with the previous biochemical data available and endorse the usefulness of said monoclonal antibodies for the identification of tumour cells by means of immunohistochemical methods and, therefore, their usefulness as a tool in methods for the diagnosis of cancer, the prognosis of its evolution or the evaluation of the response to a treatment. For this reason, the scope of the invention comprises *in vitro* methods for the diagnosis of the presence of cancer in a patient, the prognosis of its evolution, the follow-up of said evolution or the evaluation of the response to a treatment in which at least one of the monoclonal antibodies of the invention is used for the detection and/or quantification of the human choline kinase alpha protein; the studies using these methods may be both retrospective and prospective. Use of antibodies AD1, AD2, AD4, AD5, AD7, AD13 and AD14 is preferred and, among these, antibodies AD1, AD4, AD5, AD7 and AD13 are especially preferred, since they were the ones to exhibit the greatest sensitivity in the assays performed. In performing these methods, it is preferred that the samples to be analysed are tissue samples taken from a human bieng, especially those from the breast, colon, lung or bladder.

On the other hand, in the therapeutic field, given the usefulness of specific ChoKα inhibitors in anti-tumour strategies, those monoclonal antibodies which interfere with choline kinase activity may be useful as specific inhibitors of said activity and, consequently, as active anti-tumour compounds. Example 2 shows the capacity of monoclonal antibodies of the invention AD3, AD8 and AD11 to inhibit the enzymatic activity of human choline kinase alpha. For this reason, the use of at least one monoclonal antibody selected from AD3, AD8 and AD11 for the manufacturing of a drug designed as anti-cancer therapy is another aspect of the invention.

As has been discussed, those compositions comprising at least one of antibodies AD1, AD2, AD3, AD4, AD5, AD6, AD7, AD8, AD9, AD10, AD11, AD12, AD13 and AD14 or combinations thereof are also an object of the invention. The antibodies present in these compositions may be bound to different substances or particles, which may be substances or particles that facilitate the detection thereof or substances or particles that facilitate the extraction of the antibody from the medium it is in, generally in order to isolate the complexes formed following their reaction with the antibody. Examples of substances that facilitate detection of the antibody include a radioactive molecule, a chromophore, a fluorophore or an enzyme that catalyses a reaction leading to an easy-to-detect product (such as a radioactive molecule, a chromophore or a fluorophore) or that starts from a compound whose reduction in concentration is also easy to detect (which may likewise be a radioactive molecule, a chromophore or a fluorophore). Typical examples of substances or particles that facilitate extraction of the antibody from the medium it is in are metallic or magnetic particles, which allow for the isolation of the molecules coupled thereto using magnetic fields; another example of such substances or particles are molecules having a high affinity for another, which makes it possible for the antibody to bind to some type of matrix or solid medium whereto the second molecule for which the molecule bound to the antibody exhibits a high affinity is bound.

Other preferred embodiments of the compositions of the invention include those which additionally comprise a second antibody that is capable of binding to at least one antibody of the invention present in the composition of the invention. The preferred second antibodies are those which interact with the Fc fragment of the antibodies of the invention, so that they do not interfere with the bond to the human choline kinase alpha antigen. The second antibody may have any origin; it may be an anti-mouse-IgG antibody generated in a goat, such as that used in the examples described further below, or generated in any other species. Preferably, the second antibody will be bound to another substance or particle, which may be a substance that facilitates the detection thereof or a substance or particle that facilitates the extraction of the antibody from the medium it is in, generally in order to isolate the complexes formed following their reaction with the antibody. Examples of these substances or particles are the same as those mentioned in the case of the possible substances or particles bound to the antibodies of the invention.

Finally, another object of the invention is a kit for the diagnosis of cancer, the prognosis of its evolution, the follow-up of said evolution or the evaluation of an anti-cancer therapy which comprises at least one of the monoclonal antibodies of the invention. The preferred kits are those comprising a second antibody capable of binding to at least one antibody of the invention present in the kit of the invention. As in the case of the compositions of the invention, those second antibodies are preferred which interact with the Fc fragment of the antibodies of the invention. The second antibody may have any origin; it may be an anti-mouse-IgG antibody generated in a goat, such as that used in the examples described further below, or generated in any other species. Preferably, the second antibody will be bound to another substance or particle, which may be a substance that facilitates the detection thereof or a substance or particle that facilitates the extraction of the antibody from the medium it is in.

The invention will now be explained in greater detail thanks to the Examples and the Figures that appear below.

### EXAMPLES

The examples below describe the procedures followed for the generation of the monoclonal antibodies, their characterisation and the assay of their capacity to be used in immunohistochemical tests. The details are the following:

### Example 1.- Generation of monoclonal antibodies

Generation of the monoclonal antibodies was performed in the following stages:

### 1.1.- Preparation of the antigen

Expression and purification of the antigen for which antibodies wish to be generated, human ChoKα, were performed as previously described, in the form of fusion protein GST-ChoKα (5,4,15). Briefly, the cDNA that encodes human ChoKα was subcloned in a prokaryote expression vector, pGEX-4T2, a vector that has the encoding sequence of glutathione-S-transferase, in such a way that the expression of said vector leads to a GST-ChoKα fusion protein. The presence of the GST protein, which is capable of binding to a semi-solid phase of sepharose bound to glutathione, facilitates semipurification of the fusion protein from the remaining bacteria proteins. For this reason, a standard procedure was followed for purification by means of the glutathione-sepharose method, according to the manufacturer's instructions (Amersham Biosciences Europe, Germany). Once ChoKα was released from GST by means of proteolysis with thrombin, the partially purified ChoKα was resolved by means of protein electrophoresis in polyacrylamide gels with SDS (SDS-PAGE). The area containing the band pertaining to ChoKα was extracted from the gel and the gel fragment extracted was homogenised in PBS with Freund's adjuvant, either complete or incomplete (supplied in both cases by Sigma-Aldrich, MO, U.S.) (1:1, volume:volume), depending on the immunisation phase in which it was desired to use the emulsion obtained.

### 1.2.- Immunisation of the mice

Six 8-week-old mice, pertaining to the Balb/c strain, were immunised by intraperitoneal route (i.p.), using the following calendar:
- Day 1: i.p. injection of 0.5 ml of antigen homogenate containing 100 µg of recombinant ChoKα purified with complete Freund's adjuvant.
- Day 15 (booster dose 1): i.p. injection of 0.5 ml of antigen homogenate containing 100 µg of purified recombinant ChoKα, but, in this case, mixed with incomplete Freund's adjuvant.
- Day 30 (booster dose 2): i.p. injection of 0.5 ml of antigen homogenate containing 100 µg of purified recombinant ChoKα mixed with incomplete Freund's adjuvant.
- Day 40 (testing of the generation of anti-ChoKα antibodies in the mice's serum). The mice exhibiting a positive immune response to ChoKα were identified by means of Western blot towards the partially purified protein, as shown in part A of Figure 1. Among the mice, mice 2 and 4 (2 for intravenous injection and 4 for intraperitoneal injection) were selected for the fusion, since they were the ones to exhibit the lowest number of unspecific bands.
- Day 42 (booster dose 3): i.v. injection (mouse 2) or i.p. injection (mouse 4) of 0.5 ml of antigen homogenate containing 100 µg of purified recombinant ChoKα mixed with incomplete Freund's adjuvant.
- Day 45: Fusion.

### 1.3.- Fusion

The selected mice were prepared by means of the fusion of splenocytes 3 days following the last booster dose. The mice's spleens were aseptically removed and placed on a plate containing 10 ml of serum-free Eagle's medium modified by Dulbecco (DMEM); disruption of the tissue was conducted until most of the cells were released and, subsequently, the splenocytes were collected, discarding the larger pieces of tissue (16). For each fusion, a total of 10⁸ splenocytes and 10⁷ NS-1 myeloma cells were used, which were cultured in a medium supplemented with 10% serum under standard temperature and humidity conditions, and with 5% CO₂. After incubating them for 24 hours, the cells were transferred to a fresh medium supplemented with 10% FBS at a final concentration of 5 x 10⁶ cells/ml. For fusion of the cells, 1 ml of serum-free DMEM containing 0.5 g of PEG 1500 (Roche) at 50°C was used.

### 1.4.- Selection, screening and cloning of hybrid colonies

The hybrids of interest were selected by keeping the cells in a HAT medium supplemented according to the manufacturer's recommendations (GIBCO, Invitrogen, California, U.S.) for 15 days; in this medium, all those cells that do not correspond to hybrid molecules of the two populations the fusion has been performed with suffer cell death. The hybridomas were cloned using the limit-dilution technique, whereof two consecutive growth rounds were performed.

In the third week following the fusion, 149 hybrid colonies were screened, testing to see if they produced antibodies. Due to its simplicity and the large number of samples that may be checked at once, selection of the positive hybrid colonies with regard to the production of immunoglobulins was performed by means of ELISA. The ELISA details are described below:
Microtitration multi-well plates (NUNC, Germany) were coated using 50 µl/well of recombinant ChoKα at 5 µg/ml or GST in distilled water. After incubating for one hour at 37°C, the plates were washed with 0.05% PBS/Tween 20, and they were blocked with 10% milk in PBS, overnight at 4°C. The plates were washed and incubated with 50 µl/well of supernatant for 1 hour at 37°C. The wells were washed and a goat anti-mouse secondary antibody with HRP (radish peroxidase) (Dako Cytomation, California) was added, at a 1:1000 dilution. The plates were incubated for 1 hour at 37°C, they were washed and developed using a solution with the ABTS colorimetric substrate (BIO-RAD, California). The reaction was stopped by adding 100 µl of 0.5% SDS.

Most of the hybrid colonies' culture supernatants exhibited a positive reaction to the ChoKα antigen, but only those colonies showing a reaction with a stronger colour than the polyclonal antibody were selected to continue with the analysis thereof. Thus, 19 colonies of hybridomas were selected and cloned using the limit-dilution technique. In order to ensure that the culture was monoclonal, two consecutive subcloning rounds were performed on a 96-well plate. A total of 49 positive clones were obtained, which were used in the assays for antibody production.

### 1.5. Clone selection

Selection of the positive clones with regard to the production of immunoglobulins was performed by means of two consecutive methods: ELISA and immunostaining in cytospin. The ELISA was performed in an analogous manner to that described in section 1.4, in order to confirm the results obtained during the cloning process. Of the 49 initial clones in which production of anti-ChoKα antibodies had been confirmed, 14 clones were selected that strongly recognised the antigen by means of ELISA; these were called AD1-AD14. An example of the selection ELISA, that pertaining to the hybridoma of antibody AD1, is shown in part B of Figure 1.

In order to obtain additional confirmation of these results and to ensure that the selected antibodies were able to recognise ChoKα under native conditions, immunostaining in cytospin was performed; this technique is based on the centrifugation of cells on a slide using a cassette and a special rotor (oscillating), such that the cells remain adhered to the slide as complete cells that do not suffer alterations and maintain their original structure. To this end, human HEK293 embryo kidney cells transitorily transfected with the expression vector of the ChoKα gene's encoding sequence were used as a positive control and, as a negative control, cells transfected with the pCDNA3b empty vector were used; both cell lines had been cultured in DMEM medium supplemented with 10% serum under standard temperature and humidity conditions, and with 5% CO₂. To this end, a total of 2.5 x 10⁴ transfected HEK293 cells were placed on a slide, loaded on a cytospin tube and centrifuged at 800 rpm for 3 minutes. In this case, the transfected cells adhere to the slide due to the centrifugation thereof (cytospin), which makes it possible to perform an immunodetection (immunostaining) assay with them, using the hybridomas' culture medium as primary antibody. After blocking the unspecific binding sites with a peroxidase blocking solution, the slides containing the adhered cells were incubated in the hybrid colonies' supernatants for 30 minutes at ambient temperature. Following two washes with PBS, an anti-mouse secondary antibody was applied for 1 hour at ambient temperature and was developed by means of standard procedures using DAB. An example of the results obtained is shown in part C of Figure 1.

The results of the immunodetections performed on cytospins confirmed that all 14 selected hybridomas produced antibodies that efficiently recognise ChoKα under native conditions. Thus, a total of 14 different clones were selected, all of them resulting from the fusion performed with cells from mouse 4, which turned out to be positive with regard to the production of anti-ChoKα immunoglobulins using both screening methods. Tests were conducted with these clones in order to characterise them.

### Example 2.- Characterisation of the monoclonal antibodies generated

Characterisation of the hybridomas generated and of the monoclonal antibodies which they produce was performed using the following techniques:
- isotyping
- Western blot
- immunoprecipitation

The results obtained are shown in Table 1.

**Table 1.- Characterisation of monoclonal antibodies AD1-AD14**

| **MoAb** | **WB^{1,*}** | **IP^{2,*}** | **Post-IP molecular activity*** | **Isotype** | **Cross-reactivity with the mouse protein** |
|---|---|---|---|---|---|
| **AD1** | ++ | ++ | +++ | IgG1. | No |
| **AD2** | ++ | ++ | ++ | IgG1. | No |
| **AD3** | +++ | ++ | - | IgG1. | No |
| **AD4** | +++ | ++ | +++ | IgG1. | No |
| **AD5** | +++ | ++ | +++ | IgG1. | No |
| **AD6** | +++ | - | - | IgG1. | Yes |
| **AD7** | +++ | ++ | +++ | IgG1. | No |
| **AD8** | +++ | ++ | - | IgG1. | No |
| **AD9** | +++ | - | - | IgG1. | Yes |
| **AD10** | +++ | - | - | IgG1. | No |
| **AD11** | +++ | + | - | IgG1. | Yes |
| **AD12** | +++ | - | - | IgG1. | No |
| **AD13** | +++ | ++ | +++ | IgG1. | No |
| **AD14** | - | ++ | ++ | IgG2a. | No |

| | | | | | |
|---|---|---|---|---|---|
| ¹ WB: detection of proteins transferred to membranes ² IP: immunoprecipitation *: Analysis in which an arbitrary quantification system was applied, wherein the reactivity obtained was classified into the following categories: +++: excellent ++: good +: low -: no detectable reaction | | | | | |

The following are the execution details for each of the tests:

### 2.1.- Isotyping

Determining the class and the subclass of the antibodies is useful to predict the antibodies' properties and is necessary in order to use them appropriately. Knowledge of these properties facilitates the correct selection of the secondary antibody that recognises the monoclonal antibody produced, or of the type of resin that effectively binds to the antibody. Moreover, it is a first indication of monoclonality (17).

According to the immunisation scheme followed, one would expect an immune response in the mice treated in which the antibodies produced were primarily of the G-immunoglobulin class (18). IgG-type antibodies often exhibit a greater affinity for antigens than other classes generated in a primary response. As shown in Table 1, the heavy chains of the monoclonal antibodies generated are γ chains of subclass 1, except in the case of monoclonal antibody AD14, which belongs to subclass 2a; these heavy chains all form antibodies of the G-immunoglobulin class. The light chains all pertained to class κ. Consequently, subsequent experiments used an anti-mouse-IgG antibody as the secondary antibody.

### 2.2.- Western blot analysis (WB)

Immunoassays performed on proteins transferred to membranes are essential to establish the true specificity of the antibodies with respect to the protein of interest (17,18). Consequently, the potential use of the monoclonal antibodies produced was investigated on Western blots (WB). To do so, HEK293 cells were transfected with the plasmid containing the encoding sequence of the human ChoKα gene or with an empty vector as a negative control. Samples of cultures of these cells were homogenised and lysed in a buffer containing 1.5 mM MgCl₂, 0.2 mM EDA, 0.3 M NaCl, 25 mM HEPES, pH 7.5, 20 mM β-glycerophosphate and 0.1% Triton X-100 with a cocktail of protease inhibitors; hereinafter, this solution will be referred to as lysis buffer. Equal quantities of cell lysates (30 µg) were separated by electrophoresis in 10% polyacrylamide gel with SDS (SDS-PAGE) as previously described (5), and the proteins were transferred to a nitrocellulose membrane. The specific bond was detected using an ECL chemoluminescence detection kit (Amersham) following the manufacturer's instructions. Each culture medium pertaining to the 14 selected hybridomas was separately incubated and a polyclonal anti-serum against human ChoKα, generated as previously described (1), was used as a positive control. 13 of the 14 selected media turned out to be positive. Part A of Figure 2 shows a representative experiment in this assay. No cross-reactions or unspecific bands were observed, which indicates the antibodies' specificity.

In addition, assays were performed to determine whether the monoclonal antibodies generated were capable of recognising mouse ChoKα (mChoK). To this end, NIH3T3 fibroblasts which overexpressed mChoK in a stable manner (NIH3T3-Tmar-9 and NIH3T3-Tmar-11 cells) were used; they were kept in a DMEM medium supplemented with 10% serum under standard temperature and humidity conditions, and with 5% CO₂. Lysates of these cells were produced in a manner analogous to that just described for the Western blot assay of the HEK293T cells (transfected), and they were likewise resolved using the SDS-PAGE technique, by transferring the proteins to nitrocellulose membranes, which were incubated with the 14 culture media. The experiment showed that, although there is a 77% structural similarity between human and mouse choline kinase (19), only monoclonal antibodies AD6, AD9 and AD11 were capable of effectively binding to mChoK in the immunoassay performed on proteins transferred to nitrocellulose membranes. The results obtained with two of these antibodies, AD6 and AD9, are shown in part B of Figure 2.

### 2.3.- Immunoprecipitation

Immunoprecipitation (IP) is one of the most widely used immunochemical techniques. In order to characterise the capacity of the generated antibodies to immunoprecipitate ChoKα, micropearls of G-Sepharose protein were coated with each of the monoclonal antibodies generated and their capacity to induce immunoprecipitation of the ChoKα present in transfected HEK293 cell lysates was tested. To this end, the culture medium of each hybridoma was diluted to 1:100 in lysis buffer and incubated with micropearls coated with G Protein blocked with BSA (Sigma-Aldrich, MO) for 1 hour at 4°C. Following three washes in lysis buffer, the cell lysates were immunoprecipitated with the monoclonal antibodies bound to the micropearls.

In order to quantify the relative capacity of the monoclonal antibodies produced to precipitate ChoKα, a radioactive *in vitro* assay of choline kinase enzymatic activity in the immunoprecipitates was performed, based on the phosphorylation of choline to produce o-phosphocholine (P-Cho), as previously described (12,14), in the presence of methyl-[¹⁴C]-choline chloride (50-60 µCi/mmol, Amersham Biosciences Europe, Germany) at 37°C for 30 minutes. The samples were resolved by means of thin-layer chromatography (TLC) and ¹⁴C-PCho was quantified. Since the only source of ChoK activity is the immunoprecipitated fraction, if the antibody has been effective in the immunoprecipitation, there will be detectable ChoK activity and PCho will be generated.

As shown in Table 1, a total of 7 of the 14 monoclonal antibodies analysed (AD1, AD2, AD4, AD5, AD7, AD13, AD14) generated immunoprecipitated ChoKα in such a way that it was functional and the immunoprecipitated enzyme was completely active. However, interaction of some of the antibodies with ChoKα could interfere with their enzymatic activity and, albeit capable of immunoprecipitating the ChoKα protein, lead to a negative value in the enzymatic activity assay.

In order to evaluate the latter possibility, the capacity to immunoprecipitate ChoKα was also determined by means of Western blot using the polyclonal antibody to react with the potential ChoKα immunoprecipitated by the monoclonal antibodies that gave negative results in the enzymatic assay. As shown in Table 1, monoclonal antibodies AD3 and AD8 and, to a lesser extent, AD11, were capable of immunoprecipitating ChoKα. These results suggest that some of the monoclonal antibodies may interfere with ChoKα's enzymatic activity, giving rise to the absence of ChoK activity in the immunoprecipitates.

### Example 3.- Immunohistochemical tests

Finally, the potential usefulness of the antibodies generated for immunohistochemistry (IHC) was analysed. The monoclonal antibodies were first assayed in cell cytospins soaked in paraffin which overexpressed ChoKα under optimal experimental conditions. In order to ensure high concentrations of the antigen, HEK293 cells were transfected with the plasmid encoding ChoKα or with the pCDNA3b empty vector as a negative control, and the cells were cultured in DMEM medium supplemented with 10% serum under standard temperature and humidity conditions, and with 5% CO₂.

About 10 million HEK293T cells (transfected) which overexpressed human ChoKα were centrifuged by means of the cytospin technique and the sedimented cells were soaked in liquid paraffin, following a procedure analogous to that usually performed on tissue sections, both in research laboratories and in pathological anatomy services in various hospitals. After 24 hours of fixation in formalin/acetic acid/alcohol (FAA), the cell sediments were dehydrated starting with 50% ethanol for 30 minutes and gradually increasing the alcohol concentration to 100%. The sediments were introduced in xylene after their inclusion in liquid paraffin, in order to clear them.

Subsequently, cytoimmunostainings with the monoclonal antibodies of the invention were performed, and a strong specific cytoplasmic labelling was obtained in transfected HEK293T/ChoKα cells. These results indicate that the monoclonal antibodies generated are capable of recognising ChoKα also in paraffin-soaked cells, showing a high affinity for ChoKα.

Since ChoKα has been recently described as a new oncogene (4) with a high incidence in human cancer (5-7), the potential use of the monoclonal antibodies in cancer diagnosis was also assayed by means of immunohistochemical (IHC) analysis of human samples, by performing staining immunohistochemical tests of ChoKα in a few biopsies from patients of the La Paz Hospital (Madrid, Spain) who had previously been diagnosed with breast cancer and which had been stored soaked in paraffin following the standard procedure. The paraffin blocks of the different samples were cut in 5-µm sections. Subsequently, the sections were de-paraffinised and the endogenous peroxidase activity was blocked by incubation in 3% H₂O₂ in methanol for 10 minutes at ambient temperature. The antigens were recovered by incubation in 940 µM EDTA, pH 7.2, for 45 minutes at 155°C. The anti-ChoK primary monoclonal antibody was diluted to 1:2 in TBS with 1% BSA. The tissue sections were incubated for 1 hour at ambient temperature. The sections were immersed in TBS and incubated with the peroxidase-based EnVision^{™} kit (Dako Cytomation, CA) for 30 minutes at ambient temperature. The samples were subsequently incubated with the chromogenic substrate diaminobenzidine (Dako Cytomation, CA) for 5 minutes at ambient temperature. The sections were counterstained with haematoxylin.

Figure 3 shows the results obtained with samples taken from colorectal tumours in different patients, wherein one can observe an example of an antibody that is not valid for immunohistochemistry, AD9 (negative staining), an antibody that is not recommendable for use in this technique since it exhibits a pattern of weak staining, AD6, and two examples of antibodies, AD6 and AD1, which are considered to be good for use in immunohistochemistry, since they lead to strong staining of the tumoural tissue and low baseline staining of the adjacent normal tissue.

Figure 4 shows the results obtained with antibody AD1 (considered to be valid for analysis of the expression of ChoKα by immunohistochemistry) in biopsies from cancerous breast tissue (second row, labelled with number 2), as well as in biopsies pertaining to patients who had been diagnosed with lung cancer, which included NSCLC and SCLC cancer samples (first row, labelled with number 1). Analysis of the stained biopsies shows strong labelling of some of the tumoural tissues, while the adjacent normal tissue exhibits faint staining. However, in some of the biopsies both the tumoural cells and the normal tissue cells showed baseline ChoKα staining. These results are consistent with previous studies by the authors of the invention, in which 40-60% of breast, lung, colon and prostate tumours showed an increase in ChoKα levels or choline kinase activity.

Among the 14 monoclonal antibodies, 10 of them led to immunostaining, producing similar results in samples pertaining to the same tissues, whereas the remaining 4 gave negative results in the IHC analysis. The behaviour of each of the antibodies is summarised in Table 2.

**Table 2.- Behaviour of the monoclonal antibodies in immunohistochemical assays**

| **Monoclonal Antibody** | **Immunohistochemical reaction*** |
|---|---|
| **AD1** | +++ |
| **AD2** | +++ |
| **AD3** | +++ |
| **AD4** | ++ |
| **AD5** | +++ |
| **AD6** | + |
| **AD7** | + |
| **AD8** | + |
| **AD9** | - |
| **AD10** | + |
| **AD11** | - |
| **AD12** | - |
| **AD13** | - |
| **AD14** | ++ |

| | |
|---|---|
| ^{*}: The value given to the reactivity obtained was: +++ excellent ++ good + low -: no detectable reaction | |

### BIBLIOGRAPHICAL REFERENCES

1. Bhakoo KK, Williams SR, Florian CL, Land H, Noble MD. Immortalization and transformation are associated with specific alterations in choline metabolism. Cancer Res. 1996; 56:4630-5.
2. Nakagami K, Uchida T, Ohwada S, Koibuchi AND, Suda AND, Sekine T, Morishita AND. Increased choline kinase activity and elevated phosphocholine levels in human colon cancer. Jpn J Cancer Res. 1999 ; 90:419-24.
3. Cuadrado A, Carnero A, Dolfi F, Jimenez B, Lacal JC. Phosphorylcholine: a novel second messenger essential for mitogenic activity of growth factors. Oncogene. 1993; 8:2959-68.
4. Ramirez de Molina A, Gallego-Ortega D, Sarmentero J, Banez-Coronel M, Martin-Cantalejo AND, Lacal JC. Choline kinase is a novel oncogene that potentiates RhoA-induced carcinogenesis. Cancer Res. 2005 Jul 1;65(13):5647-53
5. Ramirez de Molina A, Gutierrez R, Ramos MA, Silva JM, Silva J, Bonilla F, Sanchez JJ, Lacal JC. Increased choline kinase activity in human breast carcinomas: clinical evidence for a potential novel antitumor strategy. Oncogene. 2002; 21:4317-22.
6. Ramirez de Molina A, Rodriguez-Gonzalez A, Gutierrez R, Martinez-Pineiro L, Sanchez J, Bonilla F, Rosell R, Lacal J. Overexpression of choline kinase is a frequent feature in human tumor-derived cell lines and in lung, prostate, and colorectal human cancers. Biochem Biophys Res Commun. 2002; 296:580-3.
7. Ramirez de Molina A, Banez-Coronel M, Gutierrez R, Rodriguez-Gonzalez A, Olmeda D, Megias D, Lacal JC. Choline kinase activation is a critical requirement for the proliferation of primary human mammary epithelial cells and breast tumor progression. Cancer Res. 2004; 64:6732-9
8. Rodriguez-Gonzalez A, Ramirez de Molina A, Fernandez F, Lacal JC. Choline kinase inhibition induces the increase in ceramides resulting in a highly specific and selective cytotoxic antitumoral strategy as a potential mechanism of action. Oncogene. 2004; 23:8247-59.
9. Rodriguez-Gonzalez A, Ramirez de Molina A, Banez-Coronel M, Megias D, Lacal JC. Inhibition of choline kinase renders a highly selective cytotoxic effect in tumour cells through a mitochondrial independent mechanism. Int J Oncol. 2005; 26:999-1008.
10. Ramirez de Molina A, Rodriguez-Gonzalez A, Penalva V, Lucas L, Lacal JC. Inhibition of ChoK is an efficient antitumor strategy for Harvey-, Kirsten-, and N-ras-transformed cells. Biochem Biophys Res Commun. 2001; 285:873-9.
11. Hernandez-Alcoceba R, Saniger L, Campos J, Nunez MC, Khaless F, Gallo MA, Espinosa A, Lacal JC. Choline kinase inhibitors as a novel approach for antiproliferative drug design. Oncogene. 1997; 15:2289-301.
12. Hernandez-Alcoceba R, Fernandez F, Lacal JC. In vivo antitumor activity of choline kinase inhibitors: a novel target for anticancer drug discovery. Cancer Res. 1999; 59:3112-8.
13. Rodriguez-Gonzalez A, de Molina AR, Fernandez F, Ramos MA, Nunez M del C, Campos J, Lacal JC. Inhibition of choline kinase as a specific cytotoxic strategy in oncogene-transformed cells. Oncogene. 2003; 22:8803-12.
14. Ramirez de Molina A, Penalva V, Lucas L, Lacal JC. Regulation of choline kinase activity by Ras proteins involves Ral-GDS and PI3K. Oncogene. 2002 Jan 31;21(6):937-46.
15. Hosaka K, Tanaka S, Nikawa J, Yamashita S. Cloning of a human choline kinase cDNA by complementation of the yeast cki mutation. FEBS Lett. 1992; 304:229-32.
16. Köhler G. and Milstein C. Continuous cultures of fused cells secreting antibody of predefined specificity. Nature, 256:495, 1975.
17. Hurrell J.G.R. Monoclonal Hybridoma Antibodies: Techniques and Applications, CRC Press, Boca Raton, FL, 1985, 240.
18. Harlow, E and Lane, D. 1988. Antibodies: A Laboratory Manual. Cold Spring Harbor Laboratory, Cold Spring Harbor, N.AND.
19. Altschul, Stephen F., Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs". Nucleic Acids Res. 25:3389-3402.

## Claims

1. A specific monoclonal antibody for the human choline kinase alpha protein, which is selected from the group formed by AD1, AD2, AD3, AD4, AD5, AD6, AD7, AD8, AD9, AD10, AD11, AD12, AD13 and AD14.

2. A hybridoma cell line capable of producing a specific monoclonal antibody for the human choline kinase alpha protein, which is selected from the group formed by AD1, AD2, AD3, AD4, AD5, AD6, AD7, AD8, AD9, AD10, AD11, AD12, AD13 and AD14.

3. Hybridoma cell line, according to claim 2, produced by fusion of the mouse NS-1 myeloma line with spleen cells from Balb/c mice immunised with the human choline kinase alpha protein.

4. A composition comprising at least one specific monoclonal antibody for the human choline kinase alpha protein, which is selected from the group formed by AD1, AD2, AD3, AD4, AD5, AD6, AD7, AD8, AD9, AD10, AD11, AD12, AD13 and AD14, or combinations thereof.

5. Composition according to claim 3, wherein the monoclonal antibody is bound to another substance or particle.

6. Composition according to claim 5, wherein the monoclonal antibody is bound to a substance that facilitates its detection.

7. Composition according to claim 6, wherein the monoclonal antibody is bound to a radioactive molecule, a chromophore, a fluorophore or an enzyme that catalyses a reaction wherein a chromophore or a fluorophore is produced or wherein a chromophore or a fluorophore disappears.

8. Composition according to claim 5, wherein the monoclonal antibody is bound to a substance or particle that facilitates its extraction from the medium it is in.

9. Composition according to claim 8, wherein the monoclonal antibody is bound to a metallic particle or a magnetic particle.

10. Composition according to claim 4, which additionally comprises a second antibody that is capable of binding to at least one monoclonal antibody selected from the group formed by AD1, AD2, AD3, AD4, AD5, AD6, AD7, AD8, AD9, AD10, AD11, AD12, AD13 and AD14 that is present in said composition.

11. Composition according to claim 10, wherein the second antibody is an antibody that binds to the Fc fragment of mouse G-immunoglobulins.

12. Composition according to claim 10 or 11, wherein the second antibody is bound to a substance or particle that facilitates its detection or facilitates its extraction from the medium it is in.

13. Composition according to claim 12, wherein the second antibody is bound to a radioactive molecule, a chromophore, a fluorophore or an enzyme that catalyses a reaction wherein a chromophore or a fluorophore is produced or wherein a chromophore or a fluorophore disappears.

14. Composition according to claim 12, wherein the second antibody is bound to a metallic particle or a magnetic particle.

15. Use of at least one antibody from claim 1, or a composition from claims 4 to 14, that contains it in order to detect and/or quantify the human choline kinase alpha protein in any given sample.

16. Use according to claim 15, wherein the detection and/or quantification of the human choline kinase alpha protein in any given sample is performed by means of an immunoassay wherein the proteins present in the sample to be analysed have previously been subjected to a Western blot assay.

17. Use according to claim 16, wherein the monoclonal antibody used in the immunoassay is selected from the group formed by AD1, AD2, AD3, AD4, AD5, AD6, AD7, AD8, AD9, AD10, AD11, AD12, AD13 and AD14.

18. Use according to claim 15, wherein the detection and/or quantification of the human choline kinase alpha protein in any given sample is performed following immunoprecipitation thereof.

19. Use according to claim 18, wherein the monoclonal antibody used for immunoprecipitation is selected from the group formed by AD1, AD2, AD3, AD4, AD5, AD7, AD8, AD11, AD13 and AD14.

20. Use according to claim 18, wherein the detection and/or quantification of the choline kinase alpha protein following the immunoprecipitation thereof comprises a stage wherein the human choline kinase alpha activity detectable in the immunoprecipitate is assayed.

21. Use according to claim 20, wherein the monoclonal antibody used for immunoprecipitation is selected from the group formed by AD1, AD2, AD4, AD5, AD7, AD13 and AD14.

22. Use according to any of claims 16 to 21, wherein the sample is a biological sample taken from a human being.

23. Use according to claim 22, wherein the biological sample is a sample of a biological fluid taken from a human being.

24. Use according to claim 22, wherein the biological sample is a tissue sample taken from a human being.

25. Use according to claim 15, wherein the detection and/or quantification of the human choline kinase alpha protein is performed by means of immunohistochemical techniques.

26. Use according to claim 25, wherein the detection and/or quantification of the human choline kinase alpha protein is performed on a tissue sample taken from a human being.

27. Use according to claim 26, wherein the monoclonal antibody used to perform the immunohistochemical techniques is selected from the group formed by AD1, AD2, AD3, AD4, AD5, AD6, AD7, AD8, AD10, AD14.

28. Use according to claim 26 or 27, wherein the tissue wherefrom the sample has been extracted is breast, lung, colon, prostate or bladder tissue, or tissue from a haematological tumour.

29. A method to perform *in vitro* diagnosis of cancer, prognosis of its evolution, follow-up of said evolution or to check the effectiveness of an anti-cancer treatment, which comprises the detection and/or quantification of the human choline kinase alpha protein in a biological sample from a human being by means of a monoclonal antibody selected from the group formed by AD1, AD2, AD3, AD4, AD5, AD6, AD7, AD8, AD9, AD10, AD11, AD12, AD13 and AD14.

30. Method according to claim 29, wherein the biological sample is a tissue sample taken from a human being.

31. Method according to claim 30, wherein the tissue sample is breast, lung, colon, prostate or bladder tissue, or tissue from a haematological tumour.

32. Method according to claim 30 or 31, wherein the detection and/or quantification of the human choline kinase alpha protein is performed by means of immunohistochemical techniques.

33. Method according to claim 32, wherein the detection of the monoclonal antibody bound to the human kinase alpha protein is performed by means of a second antibody capable of binding to the monoclonal antibody.

34. Method according to claim 33, wherein the second monoclonal antibody capable of binding to the monoclonal antibody is bound to a radioactive molecule, a chromophore, a fluorophore or an enzyme that catalyses a reaction wherein a chromophore or a fluorophore is produced or wherein a chromophore or a fluorophore disappears.

35. Method according to claim 33 or 34, wherein the second antibody capable of binding to the specific monoclonal antibody for human choline kinase alpha is an antibody that binds to the Fc fragment of mouse G-immunoglobulins.

36. Method according to any of claims 32 to 35, wherein the specific monoclonal antibody for human choline kinase alpha used to perform the immunohistochemical techniques is selected from the group formed by AD1, AD2, AD3, AD4, AD5, AD6, AD7, AD8, AD10 and AD14.

37. A kit designed to perform *in vitro* diagnosis of cancer, prognosis of its evolution, follow-up thereof or to check the effectiveness of an anti-cancer treatment in a biological sample from a human being which comprises at least one monoclonal antibody from claim 1.

38. Kit according to claim 37, which additionally comprises a second antibody capable of binding to at least one monoclonal antibody from claim 1 that is present in the kit.

39. Kit according to claim 38, wherein the second antibody capable of binding to at least one monoclonal antibody from claim 1 is bound to a radioactive molecule, a chromophore, a fluorophore or an enzyme that catalyses a reaction wherein a chromophore or a fluorophore is produced or wherein a chromophore or a fluorophore disappears.

40. Kit according to any of claims 38 or 39, wherein the second antibody capable of binding to at least one monoclonal antibody from claim 1 present in the kit is an antibody that binds to the Fc fragment of mouse G-immunoglobulins.

41. Use of at least one monoclonal antibody selected from the group formed by AD3, AD8 and AD11 for the manufacturing of a drug designed for anti-cancer therapy.

42. Use according to claim 41, wherein the cancer is breast, lung, colon, prostate or bladder cancer, or a haematological tumour.
